(19) Europäisches Patentamt / European Patent Office / Office européen des brevets

(11) **EP 3 467 476 B1**

(12) **EUROPEAN PATENT SPECIFICATION**

(45) Date of publication and mention
of the grant of the patent:
**26.10.2022 Bulletin 2022/43**

(21) Application number: **17830545.4**

(22) Date of filing: **21.07.2017**

(51) International Patent Classification (IPC):
**G01N 21/78** $^{(2006.01)}$     **G01N 31/22** $^{(2006.01)}$
**B22F 1/054** $^{(2022.01)}$     G01N 21/25 $^{(2006.01)}$
G01N 21/29 $^{(2006.01)}$     B82Y 30/00 $^{(2011.01)}$
C22C 1/04 $^{(2006.01)}$     C22C 5/06 $^{(2006.01)}$
G01N 33/497 $^{(2006.01)}$

(52) Cooperative Patent Classification (CPC):
(C-Sets available)
**G01N 21/783; B22F 1/054; G01N 31/22;**
B22F 1/0545; B22F 1/16; B22F 2999/00;
B82Y 30/00; C22C 1/0466; C22C 5/06;
G01N 21/251; G01N 21/293; G01N 2033/4975

(Cont.)

(86) International application number:
**PCT/ES2017/070532**

(87) International publication number:
**WO 2018/015607 (25.01.2018 Gazette 2018/04)**

(54) **COLORIMETRIC SENSING BASED ON SILVER NANOPARTICLES FOR THE DETERMINATION OF VOLATILE SULFUR COMPOUNDS**

KOLORIMETRISCHE SENSORIK AUF DER BASIS VON SILBERNANOPARTIKELN ZUR BESTIMMUNG VON FLÜCHTIGEN SCHWEFELVERBINDUNGEN

DÉTECTION COLORIMÉTRIQUE À BASE DE NANOPARTICULES D'ARGENT POUR LA DÉTERMINATION DE COMPOSÉS VOLATILS DE SULFURE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.05.2016 ES 201600440**

(43) Date of publication of application:
**10.04.2019 Bulletin 2019/15**

(73) Proprietor: **Universitat de València**
**46010 Valencia (ES)**

(72) Inventors:
- **JORNET MARTÍNEZ, Neus**
  **46010 Valencia (ES)**
- **ARGENTE GARCÍA, Ana Isabel**
  **46010 Valencia (ES)**
- **CAMPÍNS FALCÓ, Pilar**
  **46010 Valencia (ES)**
- **MOLINS LEGUA, Carmen**
  **46010 Valencia (ES)**
- **MOLINER MARTÍNEZ, Yolanda**
  **46010 Valencia (ES)**
- **HERRÁEZ HERNÁNDEZ, Rosa**
  **46010 Valencia (ES)**
- **VERDÚ ANDRÉS, Jorge**
  **46010 Valencia (ES)**

(74) Representative: **Elzaburu S.L.P.**
**Edificio Torre de Cristal**
**Paseo de la Castellana 259 C, planta 28**
**28046 Madrid (ES)**

(56) References cited:
**WO-A1-97/05482**     **US-A1- 2010 330 703**
**US-A1- 2012 058 697**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- PANICHEV N ET AL: "Solid phase extraction of trace amount of mercury from natural waters on silver and gold nanoparticles", ANALYTICA CHIMICA ACTA, ELSEVIER, AMSTERDAM, NL, vol. 813, 13 January 2014 (2014-01-13), pages 56-62, XP028610997, ISSN: 0003-2670, DOI: 10.1016/J.ACA.2014.01.011
- RAÚL A. MORALES-LUCKIE ET AL: "Facile Solventless Synthesis of a Nylon-6,6/Silver Nanoparticles Composite and Its XPS Study", INTERNATIONAL JOURNAL OF POLYMER SCIENCE, vol. 2013, 1 January 2013 (2013-01-01), pages 1-8, XP055452458, ISSN: 1687-9422, DOI: 10.1155/2013/235850
- NEUS JORNET-MARTÍNEZ ET AL: "Nylon-Supported Plasmonic Assay Based on the Aggregation of Silver Nanoparticles: In Situ Determination of Hydrogen Sulfide-like Compounds in Breath Samples as a Proof of Concept", ACS SENSORS, vol. 4, no. 8, 31 July 2019 (2019-07-31), pages 2164-2172, XP055668732, ISSN: 2379-3694, DOI: 10.1021/acssensors.9b01019
- CHEN, RUI et al.: "Fast detection of hydrogen sulfide gas in the ppmv range with silver nanoparticle films at ambient conditions", Sensors and Actuators B: Chemical, vol. 186, 2013, pages 431-438, XP055452454,
- AKAMATSU, KENSUKE et al.: "Preparation and characterization of polymer thin films containing silver and silver sulfide nanoparticles", Thin Solid Films, vol. 359, no. 1 , pages 55-60, XP004321466,
- MORALES-LUCKIE, RAUL A. et al.: "Facile solventless synthesis of a nylon-6, 6/silver nanoparticles composite and its XPS study", International Journal of Polymer, vol. 2013, 2013, pages 1-8, XP055452458,
- SHANMUGARAJ: "Colorimetric determination of sulfide using chitosan-capped silver nanoparticles", Microchimica Acta, vol. 183, no. 5, 14 March 2016 (2016-03-14), pages 1721-1728, XP035887600,

(52) Cooperative Patent Classification (CPC): (Cont.)

C-Sets
B22F 2999/00, B22F 1/16, C22C 1/0466, B22F 1/054

**Description**

The present invention belongs to the field of detection of volatile sulfur compounds (R-S⁻and S⁻²), such as hydrogen sulfide (H2S) and methylmercaptan (CH3SH) in gases, for example air. The type of sampling is passive and detection is quick and simple, by direct visual observation of changes in colour. It enables the detection of low levels of these compounds and can be applied, inter alia, to the analysis of halitosis in human breath.

The present disclosure describes a colorimetric solid sensor for the determination of volatile sulfur compounds (R-S⁻ and S⁻²). It is based on the immobilisation of silver nanoparticles (hereinafter abbreviated as AgNPs, preferably AgNPs) stabilised with sodium citrate and, in accordance with the present invention, immobilised in a nylon membrane. This sensor does not require any type of pretreatment for use thereof, since it is a solid sensor which is used directly at the time of determination; it has sustainable characteristics and is non-toxic. Also, it stands out for its application potential and simplicity, enabling the quantitative and/or semi-quantitative determination of sulfurs by simple visual observation, with detection limits of 45 ppb (v/v) and quantification of 150 ppb (v/v) obtained by measuring colour by means of diffuse reflectance and within 10 minutes of exposure time, although increasing exposure time to 30 minutes makes it possible to achieve limits of detection of 25 ppb (v/v). It has good stability at room temperature protected by a film, remaining stable for a period of three months.

## STATE OF THE ART

Volatile sulfur compounds such as hydrogen sulfide (H2S), characterised by its rotten egg smell, are environmentally toxic and harmful gases. They are formed mainly from the decomposition of organic matter and are usually found in nature in volcanic gases, natural gas, crude oil, stagnant water, etc. However, the larger amounts of sulfur and hydrogen and other volatile sulfur compounds are generated as a consequence of industrial activities such as the processing and refining of oil/natural gas, waste water treatment plants, landfills, etc. According to Royal Decree 678/2014, which marks the objectives for improving air quality, the average concentration of hydrogen sulfide in 30 minutes shall not exceed 100 $\mu$g/m³ (83 ppb v/v).

Other relevant volatile sulfur compounds are methylmercaptan (CH3SH) and dimethylmercaptan ($(CH_3)_2S$). These compounds, together with hydrogen sulfide, are responsible for bad breath and/or halitosis. Halitosis is a common problem that affects 25% of people and it is believed that 50% of people will suffer from it at some point in their lives. For people who suffer from it, this problem can hamper their normal activity and interaction with society. At present, halitosis remains a taboo subject and research in this connection is very limited. According to several of the studies analysed (Analytica Chimica Acta 2010, 661, 97, International Oral Science 2012, 4, 55; Sensors and Actuators 2009, 136, 73; Med. Princ. Pract. 2011, 20, 75) it can be concluded that concentrations below 100-200 ppb (v/v) would fall within the range of normality, while values equal to or greater than 300-400 ppb (v/v) would produce a persistent oral smell diagnosable as severe halitosis.

A compilation of different types of methods for monitoring hydrogen sulfide can be found in Trends in Analytical Chemistry, 2012, 32, 87-99. In this compilation different types of sensors are described, from those consisting of semiconductor metal oxides to those based on surface acoustic waves.

The most widely used methods for detecting volatile sulfur compounds are gas chromatography methods (Health Science 2014, 2, 80). They are sensitive and accurate methods; however, they are very expensive and are not portable. These methods require relatively long analysis times, with multiple sample conditioning and preparation stages that can only be carried out by experienced personnel. Although they can be used to detect halitosis, specialists such as dentists and medical professionals use a halimeter, a less expensive, portable instrument that is easier to use. But this instrument is still not within reach of consumers due to its high price.

Other methods used are electrochemical sensors, wherein semiconductor metal oxides or conductor polymers are used (Trends in Analytical Chemistry, 2012, 32, 87). They are based on the absorption of sulfur by the metal oxide, producing an electrical signal that can be monitored in real time. However, in many cases they have considerable environmental stability problems.

Optical sensors have advantages in terms of their applicability and functionality; in many cases they enable real-time, in situ detection with zero energy cost. Therefore, they are usually the most widely used among the population in general due to their cost, simplicity and quick response. Lead acetate strips are one example: in the presence of sulfurs they change colour from white to grey/black, forming lead sulfide (PbS). The limits of detection are in the range of 5-10 mg/L (ppm) and their use, although frequent, is not entirely recommended, due to the toxicity of the lead, which is neurotoxic (Journal Air Pollution Control Association 1966, 16, 328). Commercial colorimetric tubes, which enable the detection of lower concentrations of hydrogen sulfide (0.2-5 mg/L) although with low reproducibility, can also be used. Colorimetric tubes require active sampling with an external energy source, which involves specific sampling equipment and an energy cost to be taken into account.

As a passive sensor, a sensor that makes it possible to determine only low concentrations of hydrogen sulfide is described in Anal. Chem. 2016, 88, 1553-1558. The sensor is a sheet of paper coated with $Bi(OH)_3$ or its alkaline derivatives at pH=11 and requires a preliminary sensor conditioning stage by addition of NaOH. In the presence of the gas, the sensor changes colour from white to yellow/brown. Although it exhibits good features, there is no data regarding its selectivity against other gases or its response to real samples or atmospheres.

The detection of hydrogen sulfide in air using gold nanoparticles with a visual detection limit of 0.5 ppm has also been described in ACS Appl. Mater Interfaces 2014, 6, 6300-6307. In this case, a sample of the gas to be analysed is made to bubble in an aqueous solution whereto the gold nanoparticles are subsequently added, which are not immobilised in any medium, incubating the solutions for a few minutes. Lastly, the solutions are subjected to UV-visible spectrometry measurements.

The use of nanoparticle films whereto metal ions have been incorporated for the detection of volatile sulfur compounds has also been described in US 2009/0140752 A1. Although many metal ions are cited for their potential use therein, however experimental testing is carried out only on films incorporating gold ions.

US 2010/0330703 A1 discloses methods of determining binding involving particles, e.g. using colorimetric and other signaling techniques.

US 2012/0058697 A1 discloses textile fibres and other fibrous substrates functionalized with particles for use in the detection of targets of interest by spectroscopic methods.

In Panichev N et al, "Solid phase extraction of trace amount of mercury from natural waters on silver and gold nanoparticles", Analytica Chimica Acta, Elsevier, Amsterdam, NL, vol. 813, 13 January 2014, pages 56-62, the authors propose silver and gold nanoparticles impregnated in nylon membrane filters as a solid phase for preconcentration of mercury from natural waters.

In Raúl A. Morales-Luckie et al, "Facile solventless synthesis of a nylon-6,6/silver nanoparticles composite and its XPS study", International Journal of Polymer Science, vol. 2013, 1 January 2013, pages 1-8, the authors disclose silver nanoparticles that are synthesized and supported on thin nylon membranes by means of a simple method of impregnation and chemical reduction of Ag ions at ambient conditions.

Lastly, the use of a colloidal silver nanoparticle solution for use in the detection of organosulfur compounds released during the decomposition of onions is described in Sensors and Actuators B 2016, 228, 471-479. The nanoparticles of this document are spherical silver balls coated with polyethylene glycol and trisodium citrate, approximately 3-4 nm in size on average, and are used in the form of colloidal suspension in a tube disposed at the gas dryer outlet where the decomposing onions are placed. In this document, the inventors did not observe visual changes in the colour of the silver nanoparticles over the next two days, but rather the coloration occurred in the days leading up to the tenth day.

Consequently, the problem to be resolved in the present disclosure is that of providing a solid colorimetric sensor for the determination of volatile sulfur compounds that will enhance the features of the sensors known in the prior art, and specifically that enables simple detection, with good sensitivity, in a portable (in situ) and passive manner in just 10 minutes. This sensor has adequate selectivity for monitoring volatile sulfur compounds and it has been observed that sam do not interfere in the detection of other volatile compounds such as amines, ethanol or acetone. Sensitivity is good, with a limit of detection and quantification of 45 ppb v/v and 150 ppb v/v, respectively, by means of diffuse reflectance. The sensor has been tested in ten healthy volunteers for the detection of bad breath, in four of which before and after ingestion of $H_2S$-rich food (garlic) as responsible for the increase in volatile sulfur levels. It has also been applied successfully to the detection of sulfurs in pipes. It is a non-toxic sensor that enables the direct detection of sulfurs in situ and in real time. Lastly, in addition to the foregoing this sensor is one of the least expensive of those available in the market for the detection of low concentrations of volatile sulfurs.

The solution to this problem is based on the fact that the inventors have discovered that it is possible to obtain a colorimetric sensor with the aforementioned advantages through the immobilisation of silver nanoparticles between 10 and 40 nm in size in a nylon membrane with a pore size of less than 8 microns, preferably between 0.22 microns and 1 micron, more preferably between 0.22 and 0.47 microns, and more preferably approximately 0.47 microns.

## SUMMARY OF THE INVENTION

The present invention is directed to a method as defined in appended independent claim 1 and to the use as defined in appended independent claim 5. Particular embodiments of the invention are defined in the appended dependent claims.

The disclosure also describes a method for manufacturing a passive colorimetric sensor for the detection and/or determination of volatile sulfurs in gases, comprising silver nanoparticles between 10 nm and 40 nm in diameter, immobilised in a nylon membrane with a pore size between 0.22 microns and 1 micron.

The disclosure also describes a manufacturing method of a passive colorimetric sensor for the detection and/or determination of volatile sulfurs in gases comprising the stage of passing a silver nanoparticle suspension through a filter containing a nylon membrane, such that the silver nanoparticles are deposited and immobilised in said membrane.

To this end, in a preferred embodiment a volume of silver nanoparticles of the required diameter is taken, for example using a plastic syringe, and is then made to pass through a filter containing the nylon membrane, where they are immobilised. If necessary or convenient, the rest of the suspension of nanparticles that are not immobilised can be made to pass through the filter again with the object of achieving a larger amount of nanoparticles therein.

**BRIEF DESCRIPTION OF THE DRAWINGS**

Fig. 1.   Explanatory diagram of the aggregation process of the silver nanoparticles of the sensor used in the invention in the presence of volatile sulfurs.

Fig. 2.   Representation of the absorbance values based on wavelength for 20 nm silver nanoparticles immobilised in membranes of A) nylon (left-hand figure), B) fibreglass (middle figure) and C) cellulose paper (right-hand figure) in $H_2S$ concentrations of a) 0 ppb (v/v), b) 250 ppb and c) 1,000 ppb (v/v). Photographs of the sensors and their response in the presence of $H_2S$ at 1,000 ppb (v/v) have been included.

Fig. 3.   Photograph of the sensors and the obtainment of the calibrated curve through the representation of the quotient between absorbance at 550 nm and at 415 nm against the logarithm of the following $H_2S$ concentrations: a) 0, b) 150, c) 250, d) 500, e) 1,000, f) 1,500 and g) 2,500 ppb v/v.

Fig. 4   A) Values obtained from the calibration curve for ten healthy volunteers. B) Values obtained for four healthy volunteers before (left-hand column) and after (right-hand column) ingesting H2S-rich food.

**DETAILED DESCRIPTION OF PREFERRED EMBODIMENTS OF THE INVENTION**

The objective of the present disclosure is that of providing a passive colorimetric sensor for the determination of low concentrations of volatile sulfur compounds applicable in the field of healthcare, detection of bad breath that may be related to periodontitis or gingivitis (Sensors and Actuators B 2009, 136, 73) and also environmental applications, control of hydrogen sulfide in critical places (waste water treatments, landfills, drains, pipes, oil processing, etc.) in compliance with the legislation established in this connection (Royal Decree 678/2014). In general, the sensor can be used for the determination of sulfur in any type of matrix wherein this type of compounds exist or are generated. To date, sufficiently sensitive and selective sensors that enable in situ and real-time monitoring have not been described.

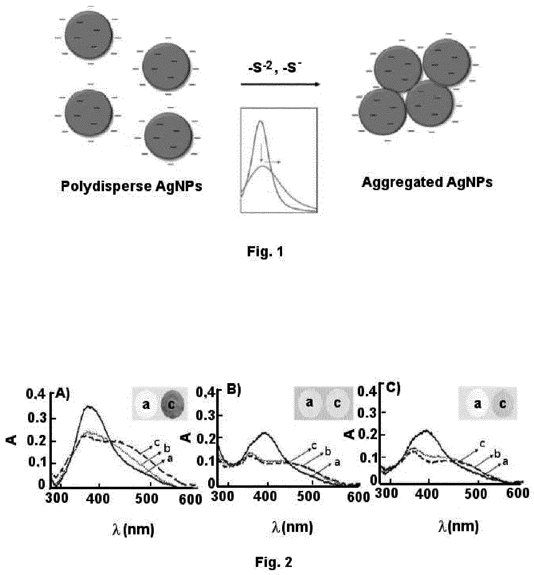

The need for this type of sensors has been resolved using silver nanoparticles immobilised in a nylon membrane. The silver nanoparticles interact with the sulfurs ($R-S^-$ and $S^{-2}$) due to the affinity of silver for sulfur. The result of this interaction gives rise to changes in colour, from yellow to ochre/brown. In the inventors' opinion, the silver nanoparticles become aggregated as a consequence of the presence of sulfur compounds, which causes the characteristic band of the polydisperse silver nanoparticles, disposed at a wavelength of 415 nm to be displaced at greater wavelengths (550 nm) and widen as the degree of aggregation thereof increases in direct relationship with the concentration of sulfurs ($-S^{2-}$ and $R-S^-$), as can be observed in the explanatory diagram shown in Figure 1. However, surprisingly this effect has been found to vary significantly depending on the substrate wherein the nanoparticles are immobilised, such that, when the immobilised in nylon membranes, a much higher retention of silver nanoparticles is obtained and the sensors also have a more intense yellow colour than when other mediums are used such as cellulose paper or fibreglass. Additionally, the optimum pore size of the nylon membrane has been determined, being between 0.22 microns and 1 micron, and more preferably between 0.22 and 0.47 microns. In the present invention it is possible to use silver nanoparticles between 10 and 40 nm in size, preferably sizes between 10 and 20 nm, due to having greater sensitivity.

This sensor has the advantages of a portable and passive sensor, due to which it does not require an external energy source or previous preparation or pretreatment. The response is obtained in just 10 minutes. In a preferred embodiment of the invention, at this point (i.e. after exposure to the sample of sulfur-containing gases) the sensors are impregnated with glycerol, the object of which is to improve the colour reading, particularly when done by visual inspection. This impregnation is preferably done by adding a few drops of glycerol (approximately 50 $\mu$L) in the centre of the sensor and spreading them with a rod or spatula, such as to distribute them over the entire surface of the sensor. Impregnation with glycerol must be done after exposing the sensor to the sulfur-containing gases, since the inventors discovered that, if done beforehand, the sensors do not show any response, which is believed to be due to the fact that AgNPs do not become aggregated under these circumstances.

One of the main problems of using silver nanoparticles as opposed to other nanoparticles such as gold nanoparticles, whose synthesis and behaviour is known in greater detail, is obtaining nanoparticles with good size dispersity. To avoid this problem, commercial nanoparticles have been used in embodiments of the invention for the purpose of avoiding the aforementioned unreproducibility problems that could stem from non-polydispersed particles, which would directly affect the sensitivity and selectivity of the sensor.

After sampling, the sensor is preferably impregnated with glycerol, obtaining an increase in colour intensity. Researchers believe that glycerol stabilises the silver nanoparticles. Impregnation with glycerol is not, however, an essential element for the invention, but rather only increases colour intensity and, consequently, process sensitivity. The invention, defined by the appended claims, could be equally carried out without the impregnation with glycerol, which would only give rise to an increase in the limits of detection and determination of the sulfurs by the sensor.

Once the sensor is coloured, the intensity of the colour can be monitored by visual inspection, by digital analysis of the red, green and blue (RGB) colur values of a photograph of a sensor obtained by means of a recording or image-capturing device such as a mobile telephone, and also by means of diffuse reflectance.

Another of the main problems of using silver nanoparticles is their stability against external factors such as light. This problem has been solved by immobilising the nanoparticles in nylon membranes; this keeps them stable for three months at room temperature.

## EXPERIMENTAL EXAMPLES

### Example 1: Sensor optimisation

As mentioned earlier, the design of the sensor is based on the immobilisation of silver nanoparticles coated with commercial citrate (Aldrich, dispersion of 0.02 mg/mL of silver nanoparticles with a particle size of 10, 20 or 40 nm (TEM) stabilised with sodium citrate in aqueous buffer) in a membrane or medium.

Different mediums were tested for the immobilisation thereof: nylon, cellulose paper and fibreglass. It was observed that the nylon has greater nanoparticle retention and the sensors had a more intense yellow colour, while the particles immobilised on the fibreglass were aggregated forming small crystals. The sensors were tested against gaseous $H_2S$ pattersn of 250 and 1,000 ppb (v/v). Only the sensor prepared in a nylon medium showed a different response for each concentration (see Figure 2).

Once the nylon membranes were selected as the most preferred medium, they were tested with different pore sizes therein. The experimental results showed that the retention of the silver nanoparticles, in addition to the sensitivity of the method, was similar for the membranes with pore sizes between 0.22 and 0.47 microns. However, in an example which does not fall within the scope of the appended claims, when membranes with a pore size of 8 microns were used retention was lower, giving rise to sensors with a lighter yellow colour, due to which it was harder to distinguish the change in colour.

Silver nanoparticles coated with citrate of different diameter sizes were immobilised: 10 nm, 20 nm and 40 nm. They all gave a positive response in the presence of different sulfur concentrations, changing from yellow to ochre and then brown. Sensitivity was similar in the case of nanoparticles with a diameter of 10 nm and 20 nm (LOD = 45 ppb), while for nanoparticles with a diameter of 40 nm worse sensitivity was observed (LOD = 200 ppb).

Various techniques were used to immobilise the silver nanoparticles in the different mediums: by deposition, by immersion and by filtering. The best results were obtained by filtering and the sensors exhibited greater colour intensity, with a much lower preparation time (a few minutes).

### Example 2: Sensor preparation

A plastic syringe (2 mL) was coupled to a plastic filter and a nylon membrane was incorporated, previously cut to the size of the filter in order for the membrane to occupy the entire filter surface. Next, between 0.2 mL and 1 mL of silver nanoparticles coated with Aldrich commercial citrate with a diameter of 20 nm and a concentration of AgNPs of 0.02 mg/mL were taken and made to pass through the filter with the nylon membrane incorporated thereto. Once the nanoparticles had passed through the filter, the excess dispersion was passed again twice through the membrane in order to retain the greatest possible amount of silver nanoparticles, achieving a retention of approximately 60% of the silver nanoparticles (0.0024 ± 0.0002 mg for the tested conditions of 0.2 mL of the dispersion taken with the syringe). Lastly, the sensors were coated with a film (parafilm) and stored at room temperature.

### Example 3: Generation of gaseous sulfur patterns

In order to evaluate sensor response to different volatile sulfur concentrations, a series of sulfur in air patterns were generated, using as a model different studies wherein the sulfur atmosphere is generated by adding an acid solution to a sodium sulfide (Na2S) or sodium methylmercaptan ($CH_3SNa$) solution. The acid added facilitates the volatilisation of the sulfur compounds.

To this end, sulfur solutions with known concentrations and an 85% phosphoric acid solution were prepared. To generate the corresponding atmosphere, 2 L static dilution bottles secured by the neck to a footstand or support by means of a clamp and on a stirring system were used. Firstly, the stirring magnet and the sensor wherethrough a string was made to pass were introduced in the bottle, such that the string was left hanging inside the bottle of static solution. Next, 0.1 mL of 85% phosphoric acid were added and the bottle was sealed. Lastly, an aqueous solution of sodium sulfur of 50 mg/L were added with a syringe through the septum of the bottle of static solution and left under stirring for 10 minutes. After the 10 minutes had elapsed, the sensor was impregnated with glycerol and its analytical response was measured by means of diffuse reflectance or the digital image of the sensor was obtained and the RGB colour analysis was performed, obtaining the corresponding values.

Example 4: Evaluation of sensor response to sulfur gas patterns

Next, sensor response to volatile compounds such as hydrogen sulfide (H2S), methylmercaptan (CH3SH) and dimethylmercaptan ((CH3)2S) was evaluated, being the hydrogen sulfide and methylmercaptan mainly responsible for halitosis. The sensor showed a positive response to the hydrogen sulfide with limits of detection and quantification of 45 ppb (v/v) and 150 ppb (v/v), respectively, while for the methylmercaptan the limits of detection and quantification were 200 ppb (v/v) and 666 ppb (v/v), respectively. No response to the presence of dimethylmercaptan was observed.

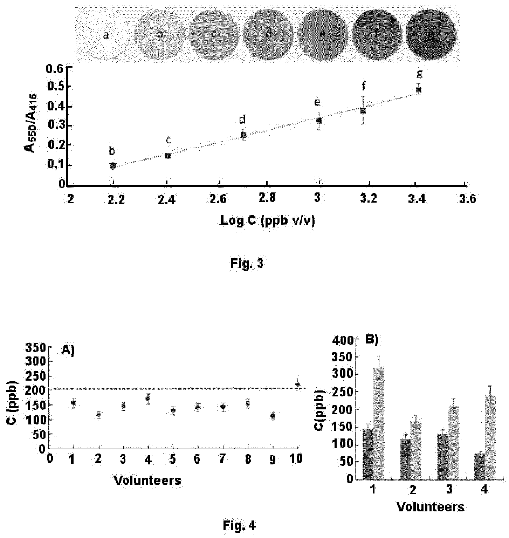

Figure 3 shows the quotient between absorbance at 550 nm (corresponding to the maximum of the aggregated nanoparticle band) and absorbance at 415 nm (corresponding to the maximum of the non-aggregated silver nanoparticles) against the logarithm of the concentration of hydrogen sulfide at 0, 150, 250, 500, 1,000, 1,500 and 2,500 ppb (v/v). Sensor response can be monitored by means of diffuse reflectance, by visual inspection as can be observed in the photograph and also by means of RGB analysis of the photograph taken using a mobile device. The calibration line obtained by means of diffuse reflectance was as follows:

$$A_{550}/A_{415} = (0.31 \pm 0.02) \, \text{Log} \, C_{\text{ppb (v/v)}} - (0.58 \pm 0.05), \, R^2 = 0.990 \quad \text{(equation 1)}$$

It was also possible to obtain a calibration line by means of RGB analysis of the photograph taken:

$$\text{RGB of the red} = (-0.056 \pm 0.018) \, C_{\text{(ppb v/v)}} + (253 \pm 3), \, R^2 = 0.991 \quad \text{(equation 2)}$$

The response to the sensors was evaluated at different exposure times. An exposure time of 10 minutes was chosen as a compromise between the intensity of the signal obtained and an adequate sampling time.

Example 5: Evaluation of sensor response in real samples

In order to evaluate whether the colorimetric sensor can be applied to the detection of halitosis, ten healthy volunteers were made to blow independently in plastic bags used for the sampling in air, wherein the sensor had been previously introduced. After 10 minutes in contact with the volunteers' breath, the sensor was extracted, impregnated with glycerol, the digital image was obtained and processed, obtaining the RGB colour coordinates and/or their colour intensity was measured by means of diffuse reflectance. The results obtained are shown in Figure 3, wherein it can be observed that the concentrations obtained fall below the concentrations considered to be persistent bad breath or severe halitosis.

Additionally, a study was carried out on the effect of the ingestion of H2S-rich food on breath. Garlic is traditionally used in Mediterranean cuisine and has very beneficial properties for the body, the most well known of which is its antibiotic power. However, the consumption of this food causes bad breath due to the presence of volatile sulfurs. In this study, four healthy volunteers were evaluated before and after ingesting a garlic-rich sauce. Figure 4B shows how the volatile sulfur levels effectively increase immediately after consuming this product.

Next, the samples were fortified, i.e. an amount of 0, 250, 300 or 500 ppb of sulfur was added thereto. To this end, volumes of 0-100 µL of a master solution of 50 mg/L of Na2S and 100 µL of 85% phosphoric acid were used in 2 L plastic bags wherein the volunteer had previously blown. After 10 minutes, the sensors were removed from the bags and impregnated with glycerol. Lastly, the digital images were obtained and/or the sensors were measured by means of diffuse reflectance.

Table 1 shows the results obtained for the recovery as a % of the aforementioned samples. The recoveries as a % were calculated as follows:

- A (column 1): Fortification or amount of sulfur (ppb) added to the samples;
- B (columns 2, 4, 6 and 8): Detection (in ppb) of the fortified sample. This concentration is obtained from the measurement of the colour of the sensors and interpolation thereof in the corresponding calibration line according to the method used, i.e. diffuse reflectance or RGB analysis of the digital images (in the calibration lines equations 1 and 2, respectively).
- C (first row at ppb=0): Concentration of sulfurs in the calculated sample based on the measurement of the colour of the sensors by means of diffuse reflectance and interpolation thereof in the calibration line which is equation 1. Values of 145 and 116 ppb were obtained for volunteer 1 and volunteer 2, respectively. The values were also calculated by means of RGB analysis of the red colour of the digital images using the computer program GIMP (equation 2). Values of 151 and 115 ppb were obtained for volunteer 1 and volunteer 2, respectively.

Therefore:

$$Recovery\ (\%) = (B\text{-}C)/A \times 100$$

For example, for a fortification of 250 ppb:

$$Recovery\ (\%) = (392\text{-}145)/250 \times 100 = 99\%$$

Table 1: Values and recoveries of two fortified samples of healthy volunteers obtained by means of diffuse reflectance and by means of RGB of the sensor photographs.

| | Volunteer 1 | | | | Volunteer 2 | | | |
|---|---|---|---|---|---|---|---|---|
| | Diffuse Reflectance | | Digital Images (GIMP) | | Diffuse Reflectance | | Digital Images (GIMP) | |
| | Detection (ppb) | Recovery (%) | Detection (ppb) | Recovery (%) | Detection (ppb) | Recovery (%) | Detection (ppb) | Recovery (%) |
| Fortified samples in (ppb) | | | | | | | | |
| 0 | 145 | - | 151 | - | 116 | - | 115 | - |
| 250 | 392 | 99 | 367 | 91 | 429 | 117 | 349 | 96 |
| 300 | 506 | 113 | 475 | 105 | 464 | 111 | 421 | 101 |
| 500 | 666 | 103 | 601 | 92 | 672 | 109 | 637 | 103 |

The results show that the colorimetric sensor of the disclosure is capable of detecting sulfurs in gases in an easy, simple and quick manner, by means of simple inspection. The sensor developed is a solid, lightweight and portable device having good limits of detection and quantification -45 ppb v/v and 150 ppb v/v, respectively, by means of diffuse reflectance-, and can be applied to the detection/control of severe halitosis. It is an environmentally friendly sensor, is completely non-toxic both for people and the environment, has a low manufacturing cost and a useful life of three months.

## Claims

1. Method for the detection and/or determination of volatile sulfurs in gases or in matrices that emit volatile sulfurs, comprising the stages of:

   a) exposing a passive colorimetric solid sensor comprising silver nanoparticles between 10 nm and 40 nm in diameter immobilised in a nylon membrane with a pore size between 0.22 microns and 1 micron, to a gas or matrix that emits volatile sulfurs for between 2 and 60 minutes;
   b) removing the sensor and determining its colouration by means of visual inspection, diffuse reflectance or RGB colour analysis of a digital image of the sensor; and
   c) based on the data obtained in stage b), determining the concentration of volatile sulfurs in the gas by means of calibration lines or colour patterns.

2. The method for the detection and/or determination of volatile sulfurs in gases according to claim 1, comprising the additional stage of impregnating the sensor with glycerol after exposure to the sulfur-containing gas but before the detection and/or determination thereof.

3. The method according to claim 2, wherein impregnation of the sensor with glycerol is carried out by depositing a few drops of glycerol on the sensor and spreading it with a spatula or rod in order to distribute it homogeneously over the entire the surface of the sensor.

4. The method according to any of claims 1 to 3, wherein the volatile sulfur-containing gases come from human breath.

5. Use of a passive colorimetric solid sensor comprising silver nanoparticles between 10 nm and 40 nm in diameter, immobilised in a nylon membrane with a pore size between 0.22 microns and 1 micron, for the detection and/or determination of volatile sulfurs in gases or in any matrix wherein volatile sulfurs are contained or are generated.

6. Use of a passive colorimetric solid sensor according to claim 5, wherein the gases come from the atmosphere or from closed or open enclosures, wherein the enclosures are selected from enclosures for processing and refining oil or natural gas, waste water treatment plants, landfills, drains and pipes.

7. Use of a passive colorimetric solid sensor according to claim 5, wherein the gases come from human breath, for the determination of halitosis.

## Patentansprüche

1. Verfahren zum Nachweis und/oder zur Bestimmung von flüchtigen Schwefelverbindungen in Gasen oder in Matrices, die flüchtige Schwefelverbindungen emittieren, die folgenden Schritte umfassend.

   a) Exposition eines passiven kolorimetrischen Feststoffsensors, der Silbernanopartikel mit einem Durchmesser zwischen 10 nm und 40 nm umfasst, die in einer Nylonmembran mit einer Porengröße zwischen 0,22 $\mu$m und 1 $\mu$m immobilisiert sind, gegenüber einem Gas oder einer Matrix, das/die flüchtige Schwefelverbindungen für einen Zeitraum zwischen 2 und 60 Minuten emittiert;
   b) Entfernen des Sensors und Bestimmen seiner Färbung mittels visueller Inspektion, diffuser Reflexion oder RGB-Farbanalyse eines digitalen Bildes des Sensors; und
   c) auf der Grundlage der in Stufe b) erhaltenen Daten Bestimmung der Konzentration von flüchtigen Schwefelverbindungen im Gas mittels Kalibrierlinien oder Farbmustern.

2. Das Verfahren zum Nachweis und/oder zur Bestimmung von flüchtigen Schwefelverbindungen in Gasen nach Anspruch 1, umfassend den zusätzlichen Schritt des Imprägnierens des Sensors mit Glycerin nach der Exposition gegenüber dem schwefelhaltigen Gas, jedoch vor dem Nachweis und/oder der Bestimmung davon.

3. Das Verfahren nach Anspruch 2, wobei das Imprägnieren des Sensors mit Glycerin durch Auftragen einiger Tropfen Glycerin auf den Sensor und Verteilen mit einem Spatel oder Stab erfolgt, um es homogen über die gesamte Oberfläche des Sensors zu verteilen.

4. Das Verfahren nach einem der Ansprüche 1 bis 3, wobei die flüchtigen schwefelhaltigen Gase aus der menschlichen Atemluft stammen.

5. Verwendung eines passiven kolorimetrischen Feststoffsensors, der Silbernanopartikel mit einem Durchmesser zwischen 10 nm und 40 nm umfasst, die in einer Nylonmembran mit einer Porengröße zwischen 0,22 $\mu$m und 1 $\mu$m immobilisiert sind, zum Nachweis und/oder zur Bestimmung von flüchtigen Schwefelverbindungen in Gasen oder in irgendeiner Matrix, in der flüchtige Schwefelverbindungen enthalten sind oder erzeugt werden.

6. Verwendung eines passiven kolorimetrischen Feststoffsensors nach Anspruch 5, wobei die Gase aus der Atmosphäre oder aus geschlossenen oder offenen Behältern stammen, wobei die Behälter aus Behältern für die Verarbeitung und Raffinierung von Öl oder Erdgas, Abwasserbehandlungsanlagen, Deponien, Abflüssen und Rohren ausgewählt sind.

7. Verwendung eines passiven kolorimetrischen Feststoffsensors nach Anspruch 5, wobei die Gase aus der menschlichen Atemluft stammen, zur Bestimmung der Halitose.

## Revendications

1. Procédé pour la détection et/ou la détermination de sulfures volatils dans des gaz ou dans des matrices qui émettent des sulfures volatils, comprenant les étapes consistant à :

   a) exposer un capteur solide colorimétrique passif comprenant des nanoparticules d'argent d'un diamètre compris entre 10 nm et 40 nm immobilisées dans une membrane de nylon ayant une taille de pores comprise entre 0,22 micron et 1 micron, à un gaz ou à une matrice qui émet des sulfures volatils pendant entre 2 et 60 minutes ;
   b) retirer le capteur et déterminer sa coloration au moyen d'une inspection visuelle, d'une réflectance diffuse ou d'une analyse de couleur RGB d'une image numérique du capteur ; et
   c) sur la base des données obtenues à l'étape b), déterminer la concentration de sulfures volatils dans le gaz au moyen de lignes d'étalonnage ou de motifs de couleur.

2. Procédé pour la détection et/ou la détermination de sulfures volatils dans des gaz selon la revendication 1, comprenant l'étape supplémentaire d'imprégnation du capteur avec du glycérol après l'exposition au gaz contenant du soufre, mais avant la détection et/ou la détermination de celui-ci.

3. Procédé selon la revendication 2, dans lequel l'imprégnation du capteur avec du glycérol est effectuée en déposant quelques gouttes de glycérol sur le capteur et en l'étalant avec une spatule ou une tige afin de le répartir de manière homogène sur toute la surface du capteur.

4. Procédé selon l'une des revendications 1 à 3, dans lequel les gaz volatils contenant du soufre proviennent de l'haleine.

5. Utilisation d'un capteur solide colorimétrique passif comprenant des nanoparticules d'argent d'un diamètre compris entre 10 nm et 40 nm, immobilisées dans une membrane de nylon ayant une taille de pores comprise entre 0,22 micron et 1 micron, pour la détection et/ou la détermination de sulfures volatils dans des gaz ou dans toute matrice dans laquelle des sulfures volatils sont contenus ou sont générés.

6. Utilisation d'un capteur solide colorimétrique passif selon la revendication 5, dans laquelle les gaz proviennent de l'atmosphère ou d'enceintes fermées ou ouvertes, dans laquelle les enceintes sont choisies parmi des enceintes pour le traitement et le raffinage du pétrole ou du gaz naturel, des installations de traitement des eaux usées, des décharges, des drains et des tuyaux.

7. Utilisation d'un capteur solide colorimétrique passif selon la revendication 5, dans laquelle les gaz proviennent de l'haleine humaine, pour la détermination de l'halitose.

**Polydisperse AgNPs**

$-S^{-2}, -S^{-}$

**Aggregated AgNPs**

**Fig. 1**

**Fig. 2**

Fig. 3

Fig. 4

## REFERENCES CITED IN THE DESCRIPTION

### Patent documents cited in the description

- US 20090140752 A1
- US 20100330703 A1
- US 20120058697 A1

### Non-patent literature cited in the description

- *Analytica Chimica Acta,* 2010, vol. 661, 97
- *International Oral Science,* 2012, vol. 4, 55
- *Sensors and Actuators,* 2009, vol. 136, 73
- *Med. Princ. Pract.,* 2011, vol. 20, 75
- *Trends in Analytical Chemistry,* 2012, vol. 32, 87-99
- *Health Science,* 2014, vol. 2, 80
- *Trends in Analytical Chemistry,* 2012, vol. 32, 87
- *Journal Air Pollution Control Association,* 1966, vol. 16, 328
- *Anal. Chem.,* 2016, vol. 88, 1553-1558
- *ACS Appl. Mater Interfaces,* 2014, vol. 6, 6300-6307
- Solid phase extraction of trace amount of mercury from natural waters on silver and gold nanoparticles. **PANICHEV N et al.** Analytica Chimica Acta. Elsevier, 13 January 2014, vol. 813, 56-62
- **RAÚL A. MORALES-LUCKIE et al.** Facile solvent-less synthesis of a nylon-6,6/silver nanoparticles composite and its XPS study. *International Journal of Polymer Science,* 01 January 2013, vol. 2013, 1-8
- *Sensors and Actuators B,* 2016, vol. 228, 471-479
- *Sensors and Actuators B,* 2009, vol. 136, 73